# EUROPEAN PATENT SPECIFICATION

(11) **EP 1 885 844 B1**
(45) Date of publication and mention of the grant of the patent: **26.05.2010**
(21) Application number: 06741127.2
(22) Date of filing: 29.05.2006
(51) Int. Cl.: C12N 5/02, C12N 5/00

(54) **PREPARATION AND USE OF BASEMENT MEMBRANE PARTICLES**
HERSTELLUNG UND VERWENDUNG VON BASALMEMBRANPARTIKELN
PREPARATION ET UTILISATION DE PARTICULES DE MEMBRANE BASALE

(30) Priority: 30.05.2005 AU 2005902758
(43) Date of publication of application: 13.02.2008
(73) Proprietor: Commonwealth Scientific and Industrial Research Organisation, Campbell, ACT 2612 (AU)
(72) Inventor: RAMSHAW, John, Alan, Maurice, Camberwell, Victoria 3124 (AU); GLATTAUER, Veronica, Ivanhoe, VIC 3079 (AU); WERKMEISTER, Jerome, Pascoe Vale, VIC 3044 (AU)
(74) Representative: Brasnett, Adrian Hugh
(86) International application number: PCT/AU2006/000708
(87) International publication number: WO 2006/128216

(56) References cited:
- EP-A1- 1 437 147
- WO-A-02/062357
- WO-A1-2005/121316
- WO-A2-2004/076631
- KOLLER M.R.: 'Cell Adhesion in Animal Cell culture: Physiological and Fluid-Mechanical Implications' BIOPROCESS TECHNOL vol. 20, 1995, pages 61 - 110, XP008073388

## Description

### FIELD OF THE INVENTION

The invention relates to the field of *in vitro* cell culture and, more particularly, to materials and methods for growing mammalian cells, such as stem cells, *in vitro.* In one particular application of the invention, the materials and methods provide a means for the *in vitro* expansion of spermatogonial stem cells. In another particular application, the materials and methods of the invention provide a means for the *in vitro* expansion of haematopoietic stem cells.

### BACKGROUND OF THE INVENTION

The ability to grow cells in culture has led to a major increase in the understanding of living processes and the development of a wide range of new products and processes, including those being developed by tissue engineering and cell transfer. Typically, mammalian cells are grown on flat surfaces such as tissue-culture polystyrene, which is polystyrene that has been modified to allow better cell attachment This surface can be further modified, for example by absorbing attachment proteins such as collagens, fibronectin, vitronectin, laminin and many other molecules. In other culturing methods, the cells may be grown on beads or particles, such as dextran beads (e.g. Cytodex beads) which are adherent for cells and which, in use, are generally gently stirred to form a suspension that allows cell growth. These methods have an advantage in that the beads or particles provide a large surface area for cell growth within a small reaction vessel, compared to the need for numerous tissue culture flasks or plates. The beads or particles used in these methods may also be modified to enhance or refine their cell attachment capability.

There has been a significant amount of work on the development of natural and synthetic substrates that allow cell attachment and proliferation, and it is now known that the type of substrate can influence the ability of a particular cell type to attach, divide and migrate. A variety of model substrates have been developed to study cell attachment. These model substrates have generally comprised a number of matrix components (e.g. collagen type I, III, IV, laminin, fibronectin, vitronectin, fibrin gel, hyaluronan, alginate and chitosan) or peptides derived from these proteins (e.g. RGD) which have been attached to tissue culture plastic, a variety of synthetic polymers, or beads.

Most cells, excepting, for example, those in the blood circulation, are attached to components of the extracellular matrix, such as collagens. For example, fibroblasts in the dermis are adherent to the collagen type I which forms the main connective tissue fibre bundles that characterise this tissue. In many cases, including, for example, endothelial cells, epithelial cells, and various types of stem cells, the preferred component for adhesion is a structure termed the basement membrane or basal lamina. Basement membranes are unique, highly organised supportive sheet-like structures in the extracellular matrix (ECM) that are formed at the interface between cells (e.g. parenchymal cells) and their surrounding tissues. There are different types of basement membranes in the body. Some act as a tissue boundary where certain cells can attach, some can act as filters with selective permeability, some support very selective cellular differentiation of a number of different cell types including stem cells, while others may act as a reservoir for growth factors. Basement membranes comprise a range of specific macromolecule components including collagen type IV, which is believed to form an extended net-like structure within the basement membrane, and a range of other components including, laminin, nidogen, entactin, agrin, bamacan, usherin and heparin sulphate proteoglycans, such as perlecan, which are believed to interact in an ordered manner with the collagen type IV structure. The most abundant components, namely collagen type IV and laminin come in a range of molecular isoforms, so particular basement membranes associated with specific tissues can vary depending upon which isoforms, or proportions of isoforms, are present. Collagen type IV, like all collagens, consists of three alpha chains wound into a coiled-coil, rope-like triple helical conformation. The first type IV collagen examined consisted of two α1[IV] collagen chains and one α2[IV] collagen chain. Subsequently, other chains have been observed, including the α3[IV] and α5[IV] chains which may substitute for the α1[IV] chain and the α4[IV] and α6[IV] chains, that may substitute for the α2[IV] chain. Various, but not all, combinations of these chains have been found in different basement membranes. Laminin consists of three different chain types, one α, one β and one γ chain. Various forms of each of these chains have been observed in different combinations, so that at least 14 different isoforms of laminin have been observed.

Thus, the addition of a substrate such as basement membrane to a culture of eukaryotic cells (e.g. stem cells), might be particularly advantageous in supporting the growth of these cells by mimicking the naturally occurring local environment ("niche") for the particular eukaryotic cells. In this regard, the present applicant has surprisingly found that particles of basement membrane can be used effectively for expanding eukaryotic cells *in vitro.* In particular, the present applicant has found that particles of a basement membrane or basement membrane-like substrate selected from the group consisting of: particles of the natural basement membrane structure; particles of an analogous structure produced in culture by selected cells; and particles of an ordered structure reconstituted from individual constituents; are suitable for the *in vitro* growth of eukaryotic cells.

Various types of basement membrane preparations have been previously described for the culturing and expansion of eukaryotic cells *in vitro;* however these differ from the particles of basement membrane or basement membrane-like substrate of the present invention, in that the basement membrane is provided in comparatively large forms or sections (e.g. basement membrane tubules or sleeves), wherein the natural, gross three-dimensional architecture of the basement membrane is essentially maintained. In contrast to the present invention, these previous basement membrane preparations are unsuitable (e.g. due to their large size or inappropriate buoyancy), for supporting the expansion of eukaryotic cells in suspension culture or other bioreactor systems. A particular example of one of these previous basement membrane preparations is described in Enders, GC et al. (1986), wherein primary Sertoli cells were adhered *in vitro* to a seminiferous tubule basement membrane (STBM) preparation comprising hollow tubes or sleeves (i.e. a preparation maintaining the gross three-dimensional architecture of the natural, tubule or sleeve form of the basement membrane). Another example is provided by Van der Wee, K and Hofmann, MC (1999), which describes the use of a soluble basement membrane component preparation (i.e. Matrigel®) coated onto tissue culture plates to support the growth of a Sertoli cell line.

### SUMMARY OF THE INVENTION

In a first aspect, the present invention provides a method for supporting the *in vitro* growth of one or more eukaryotic cell type(s), said method comprising;
seeding cells of said cell type(s) onto particles comprising basement membrane or a basement membrane-like substrate, and
culturing said seeded cells *in vitro* under conditions suitable for expansion of said seeded cells,
wherein said particles are less than about 500 µm in size.

The method is useful for the *in vitro* growth of eukaryotic cells where either maintenance of cell phenotype or, otherwise, cell differentiation (i.e. as may be induced by, for example, the addition of suitable factors) is required.

The particles utilised in the method may be particles comprising basement membrane or a basement membrane-like substrate. Particles comprising basement membrane may be prepared from natural basement membrane derived from a tissue sample of a suitable source (e.g. by extracting basement membrane from a tissue sample). Particles comprising a basement membrane-like substrate may be prepared by, for example, culturing basement membrane-synthesising cells on a solid support (e.g. suitable beads).

Thus, the method may further comprise preparing the particles onto which the said cells are seeded, by attaching onto suitable beads, basement membrane-synthesising cells and culturing the bead-bound cells under conditions suitable for the expression and secretion of basement membrane proteins to thereby produce particles comprising beads coated, at least partially, in a basement membrane-like substrate. Optionally, the basement membrane-synthesising cells may thereafter be destroyed or removed from the particles before seeding of the cells to be grown.

Alternatively, the method may further comprise preparing the particles, by attaching onto suitable beads, both the cells to be grown and basement membrane-synthesising cells, and co-culturing the bead-bound cells under conditions suitable for the expression and secretion of basement membrane proteins by the basement membrane-synthesising cells. In this way, the cells to be grown by the method become seeded onto particles comprising a basement membrane-like substrate as the beads become coated, at least partially, in the basement membrane-like substrate.

In a second aspect, the present invention provides a population of eukaryotic cells grown by a method according to the first aspect of the invention.

In a third aspect, the present invention provides particles comprising beads coated, at least partially, in a basement membrane-like substrate, wherein said particles are less than about 500 µm in size.

### BRIEF DESCRIPTION OF THE FIGURES

Figure 1 provides a photograph of particles according to the present invention prepared from basement membrane isolated from bovine seminiferous tubules according to the process described in Example 1.
Figure 2 provides a photograph of particles according to the present invention prepared from basement membrane material isolated from bovine seminiferous tubules according to the process described in Example 1 followed by fragmentation by mechanical action as described in Example 9, prior to fractionation.
Figure 3 provides a photograph of basement membrane particles, prepared in accordance with the process given in Example 1, and processed into particles by mechanical action as described in Example 9. These particles were used as cell culture substrates for spermatogonial stem culture as described in Example 14.
Figure 4 shows the presence of a basement membrane-like substrate produced from endothelial cells grown on Cytodex 3 beads. The substrate is shown by immunostaining for the presence of collagen type IV.
Figure 5 shows the presence of a basement membrane-like substrate produced from Sertoli cells grown on Cultispher-S beads, shown by immunostaining for the presence of collagen type IV.

### DETAILED DESCRIPTION OF THE INVENTION

The present applicant has found that particles of basement membrane or a basement membrane-like substrate selected from the group consisting of:
(i) particles of the natural basement membrane for the particular eukaryotic cells to be grown;
(ii) particles of an analogous structure produced in culture by selected cells, and
(iii) particles of an ordered network structure reconstituted from individual basement membrane constituents;
are suitable for the *in vitro* growth of eukaryotic cells, particularly mammalian cells.

The analogous and ordered network structures referred to in (ii) and (iii) can be regarded as particular examples of a basement membrane-like substrate. As used herein, the term "basement membrane-like substrate" refers to a structure comprising a matrix consisting of at least collagen type IV or functional fragments thereof. However, preferably, the basement membrane-like substrate used in the present invention comprises a matrix consisting of at least collagen type IV and laminin. More preferably, the basement membrane-like substrate used in the present invention comprises a network of ordered interacting molecules consisting of at least collagen type IV and laminin.

Particles according to the present invention have been found to be particularly effective for supporting the growth and proliferation of eukaryotic cells such as stem cells and, in particular, spermatogonial stem cells.

The particular application of the present invention to the growth of spermatogonial stem cells, offers considerable promise in the field of domestic animal breeding (Brinster, RL et al. (1994), and Ogawa, T et al., (1999)), such as beef breed improvement. For example, mechanisms relying on the natural breeding of cattle to propagate stock can be inefficient, so isolation and *in vitro* amplification of bovine spermatogonial stem cells from male breeding stock known to produce beef of a desirable quality, offers the possibility of readily and conveniently producing sufficient amounts of donor bovine spermatogonial stem cells suitable, for example, for transfer into other host animals to allow insemination of female cattle. Similarly, the present invention may be of considerable value in the preparation of other agriculturally valuable spermatogonial stem cells for use in breeding programs or for assisting in the rebuilding of populations of endangered species. The present invention may also be of considerable value to the generation of transgenic animals.

Particles according to the present invention have also been found to be particularly effective in supporting the growth of other types of stem cells that are found in the early embryo, foetus, placenta and umbilical cord, as well as mature tissues and organs in the body. The present invention therefore encompasses the use of particles of a natural basement membrane or basement membrane-like substrate for the *in vitro* growth of various types of stem cells.

Depending upon the source of the type of particular stem cells used (i.e. embryonic, adult mesenchymal or cord blood), the broad spectrum of cell differentiation will vary. For example, adult stem cells may have a more limited ability to differentiate into a wide range of somatic cell types (e.g. osteoblasts, chondrocytes, and adipocytes) compared with embryonic stem cells. Further, stem cells from the umbilical vein could include stem cells from cord blood, connective tissue around the cord (i.e. Wharton's jelly), as well as specialised regions like the perivascular zones in these tissues. Irrespective of the source, stem cells in general are the key cells responsible for normal growth and development and by nature, have the capacity to self renew and regenerate tissues and organs within the body.

A fundamental issue in using stem cells for cell-based transplantation, tissue engineering or regenerative medicine is the primary ability to amplify a small number of these cells to allow these cells to be re-implanted into the host to repair or regenerate damaged tissue. The present invention offers materials and methods which allow for the expansion of stem cells for use in, for example, somatic and germ cell propagation.

The invention, in a first aspect, therefore provides a method for supporting the *in vitro* growth of one or more eukaryotic cell type(s), said method comprising;
seeding cells of said cell type(s) onto particles comprising basement membrane or a basement membrane-like substrate, and
culturing said seeded cells *in vitro* under conditions suitable for expansion of said seeded cells,
wherein said particles are less than about 500 µm in size.

The method is useful for the *in vitro* growth of eukaryotic cells where either maintenance of cell phenotype (e.g. for growth of undifferentiated stem cells) or, otherwise, cell differentiation (e.g. for growth of differentiated cells from stem cells) is required. Cell differentiation may be induced by the addition to the culture of suitable differentiation factors and/ or the application of culturing conditions which encourage cell differentiation.

The particles utilised in the method are less than about 500 µm in size (i.e. the maximal dimension of the particles is less than about 500 µm in size). The particles may be irregular in shape or may have a substantially consistent shape (e.g. the particles may be substantially spheroid, wherein the diameter is less than 500 µm, or substantially ovoid, wherein the dimension of the lengthwise axis is less than 500 µm). Preferably, the particles have an average size in the range of from about 50 µm to 400 µm. More preferably, the particles have an average size of about 100 µm.

The particles may be particles comprising natural basement membrane or a basement membrane-like substrate.

Where the particles comprise natural basement membrane, the basement membrane is preferably derived from a tissue sample of a suitable source (e.g. tissue selected from, but not limited to, the group consisting of muscle, skin, kidney, seminiferous tubules of the testis, lung, placenta, bone, bone marrow, bone/bone marrow interface, blood vessels, perivascular tissue and bladder), wherein the tissue sample is unfixed. However, it is also possible to use basement membrane derived from a stabilised (i.e. fixed) tissue sample of a suitable source (e.g. as fixed using any one of the fixing agents well known to those skilled in the art, including formaldehyde, glutaraldehyde, other di-aldehydes, bis-imidates, carbodi-imides, ribose, bis- or polyepoxides, bis-isocyanates and acyl azides). It is preferable, that where the basement membrane is derived from a fixed tissue sample, the tissue sample has been fixed with glutaraldehyde or ribose.

Preferably, particles comprising a natural basement membrane are prepared by extracting the basement membrane from a tissue sample of a suitable source by treating the tissue sample with an agent such an enzyme (e.g. pepsin and/or detergent such as Triton X-100, sodium deoxycholate or N-octyl-glucoside). The extracted basement membrane is then preferably treated so as to produce particles (i.e. fragments) of intact basement membrane. This can be achieved by a number of different means, for example, the tissue may be fragmented by mechanical means such as by use of a pestle and mortar, or a mechanical blender (e.g. Polytron or Ultra Turrax (IKA Werke, Germany), a liquid nitrogen cavitation device (e.g. Parr Instrument Company), or by mechanical compression, and then fractionated by size (e.g. by use of a sieve). Various other means will be well known to those skilled in the art and are also to be regarded as encompassed within the scope of the present invention.

Where the particles comprise a basement membrane-like substrate, the basement membrane-like substrate may be selected from structures analogous to basement membrane produced in culture by selected, basement membrane-synthesising cells (i.e. cells which naturally synthesise and secrete the proteins found in basement membrane, particularly collagen type IV and laminin), and ordered structures "reconstituted" from basement membrane constituents. For example, basement membranes may be deposited on suitable beads (e.g. Cytodex or Cultispher beads) through culturing basement membrane-synthesising cells such as endothelial cells, keratinocytes, Schwann cells, Sertoli cells, osteoblasts and combinations thereof.

Thus, the method may further comprise preparing the particles onto which the said cells are seeded, the preparation comprising attaching onto suitable basement-membrane coated beads (e.g. Cytodex, cultispher beads, synthetic polymer resorbable beads/particles) which, preferably; have an average size in the range of 50 µm to 400 µm), basement membrane-synthesising cells and culturing the bead-bound cells under conditions suitable for the expression and secretion of basement membrane constituents to thereby produce particles comprising beads coated, at least partially, in a basement membrane-like substrate, having a thickness that enables cell adhesion. Optionally, the basement membrane-synthesising cells may thereafter be destroyed or removed from the particles (e.g. by detaching the cells through treatment of the particles with a dilute ammonia solution) before seeding of the cells to be grown.

Alternatively, the method may further comprise preparing the particles, by attaching onto suitable beads, both the cells to be grown and basement membrane-synthesising cells, and co-culturing the bead-bound cells under conditions suitable for the expression and secretion of basement membrane proteins by the basement membrane-synthesising cells. In this way, the cells to be grown by the method become seeded onto particles comprising a basement membrane-like substrate as the beads become coated, at least partially, in the basement membrane-like substrate.

Also, particles comprising a basement membrane-like substrate can be produced by reconstituting individual basement membrane constituents, under conditions that allow collagen type IV and laminin molecules to aggregate, to form an ordered network structure. Suitable conditions for producing particles of a basement membrane-like structure in this manner can be generated by, for example, making a 0.5 mg/ml laminin solution at least 50 micromolar with respect to calcium chloride, adding a solution of an equivalent weight of collagen type IV, then adding suitable beads (e.g. Cultispher G), and warming the mixture to 37°C with stirring.

Particles comprising a basement membrane-like substrate may be stabilised by treatment with a fixing agent such as those mentioned above, but preferably glutaraldehyde or ribose.

The basement membrane or basement membrane-like substrate comprising the particles is preferably "matched" to the cell type(s) of the cells to be grown by the method. For example, for the growth of spermatogonial stem cells, the basement membrane or basement membrane-like substrate preferably provides a niche which mimicks the natural local environment for spermatogonial stem cells found in seminiferous tubules.

Preferably, the step of culturing the seeded cells is conducted in a bioreactor. This has the particular advantage of allowing a greater proportion of cells to be grown since the culture surface area is less restricted compared with conventional "static" based tissue culture systems (e.g. culturing in tissue culture flasks).

The eukaryotic cells used in the method of the invention may be cells of a mature phenotype (e.g. endothelial cells, skin fibroblasts, my oblasts and cardiomyoblasts), or they may be cells of a primitive phenotype such as stem cells (e.g. embryonic, adult mesenchymal, or cord blood including haematopoietic stem cells).

In a particularly preferred embodiment of the present invention, the particles comprising basement membrane or a basement membrane-like substrate are used to support the *in vitro* growth of spermatogonial stem cells.

During spermatogenesis, the seminiferous tubule matrix plays a key role in orchestrating the spermatogonial stem cell survival, propagation and differentiation. The primary cell in this niche is the spermatogonia, the stem cell of the germ cell population. Spermatogonial stem cells (SCC) maintain spermatogenesis throughout life. In order to achieve this, the SCC has to both self re-new to produce further stem cells as well as differentiate along a complex pathway into mature sperm cells. To maintain or regenerate normal tissue structure and function, stem cell renewal and differentiation must be tightly regulated. In general, the Aₛ (A single) spermatagonia is classified as the stem cell. As it divides, it will either become two new independent stem cells, or remain as a pair of cells, known as A-paired (Aₚᵣ) spermatagonia. These cells can further divide into aligned arrays of 4, 8 or 16 cells, known as A-aligned (Aₐₗ) spermatagonia, which will then differentiate into A1 spermatagonia, and after six divisions will produce A2, A3, A4 and, finally, B spermatagonia that will lead to spermatocytes. Depending on the species, this classification will vary. For example, in bovine, the SCC are thought to include the Aₛ and Aₚᵣ cell types, while the A1-A4 cells are committed spermatogonial precursor cells. The nature of the true stem cell is critical for isolation and amplification of these cells for use in, for example, transplantation.

In the same niche (i.e. the seminiferous tubule matrix occupied by SCC), Sertoli cells extend from the basement membrane towards the tubule lumen. At the basement membrane, the Sertoli cells are in close or direct contact with primary spermatagonia, whereas at a distance these cells or their cell processes elongate around spermatocytes and early spermatids. In this embodiment of the invention, the particles used for growth of the spermatogonial stem cells may therefore be derived from a preparation of natural seminiferous tubules or by growth of a basement membrane or a basement membrane-like substrate produced by cells that may "deposit" a basement membrane (i.e. basement membrane-synthesising cells), particularly Sertoli cells.

In further particularly preferred embodiment of the present invention, the particles comprising basement membrane or a basement membrane-like substrate are used to support the *in vitro* growth of haematopoietic stem cells.

Adult mesenchymal stem cells and cord blood include haematopoietic stem cells (HSC) that are associated with discrete niches in the bone marrow. These niches, wherein HSC growth and differentiation is regulated, comprise endothelium-lined sinuses, feeder vessels and adipocytes, as well as an endosteal interface that comprises osteoblasts or osteoblastic cells. In addition to cell-cell signalling, these specialised niche cells can produce matrix proteins involved in cell-matrix interactions. Thus, in this embodiment, the particles used for growth of the haematopoietic stem cells may be derived from a preparation of natural basement membrane from bone marrow or by growth of a basement membrane-like substrate produced by suitable basement membrane-synthesising cells, particularly osteoblasts.

Cells grown in accordance with the method of the invention, may be removed from the particles by any of the methods well known to those skilled in the art (e.g. by treating with trypsin, versene, dispase, collagenase, hyaluronidase or combinations thereof).

In a second aspect, the present invention provides a population of eukaryotic cells grown by a method according to the first aspect of the invention.

The eukaryotic cells of the second aspect may be of a mature phenotype (e.g. endothelial cells, skin fibroblasts, myoblasts and cardiomyoblasts), but are preferably stem cells (e.g. embryonic, adult mesenchymal, or cord blood including spermatogonial stem cells and haematopoietic stem cells). More preferably, the eukaryotic cells are spermatogonial stem cells (e.g. spermatogonial stem cells selected from the group consisting of bovine, ovine, equine, feline, canine, and caprine spermatogonial stem cells) or haematopoietic stem cells (e.g. haematopoietic stem cells selected from the group consisting of human, bovine, ovine, equine, feline, canine, and caprine haematopoietic stem cells).

In a third aspect, the present invention provides a preparation of particles comprising beads coated, at least partially, in a basement membrane-like substrate, wherein said particles are less than about 500 µm in size.

In a fourth aspect, the present invention provides a preparation of particles comprising a natural basement membrane, wherein said particles are less than about 500 µm in size.

In order that the nature of the present invention may be more clearly understood, preferred forms thereof will now be described with reference to the following nonlimiting examples.

### EXAMPLES

### Example 1 Isolation of basement membrane from testis

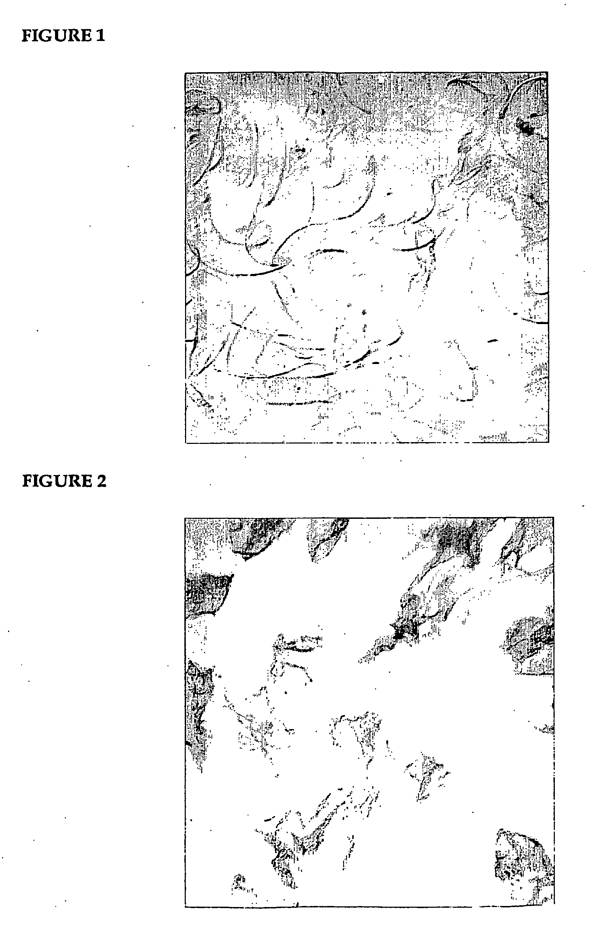

Fresh bovine testis was obtained from an abattoir. After removal of the external membranes, the tissue comprising the seminiferous tubules was sliced into approximately 5 x 5 x 5mm pieces. Pepsin (0.5 mg/ml final concentration) was added to the sliced tissue at pH 2.5 in 100 mM acetic acid and the tissue digested for between 1 and 4 days. The digested tissue was then collected by brief centrifugation, and washed several times with phosphate buffered saline (PBS) and collected by brief centrifugation or by settling. Washed samples were examined by histology using staining with Sirius red, and by immunohistology to examine the presence of basement membrane components using antibodies to laminin and collagen type IV. See Figure 1.

### Example 2 Isolation of basement membrane from kidney

Fresh bovine kidney was obtained from an abattoir. After removal of the external membranes, the tissue comprising the glomerulus was sliced into approximately 5 x 5 x 5mm pieces and treated according to the procedure given in Example 1.

### Example 3 Isolation of basement membrane from lung

Fresh bovine lung was obtained from an abattoir. After removal of extraneous tissues including the main bronchial tubes, the tissue comprising mainly the alveolar tissue was sliced into approximately 5 x 5 x 5mm pieces and treated for isolation of basement membrane particles according to the procedure given in Example 1.

### Example 4 Alternative isolation of basement membrane from testis

Fresh bovine testis was obtained from an abattoir. After removal of the external membranes, the tissue comprising the seminiferous tubule was sliced into approximately 5 x 5 x 5mm pieces and was macerated on a stainless steel wire mesh to break the tissue and release cells with continual irrigation by PBS, including a protease inhibitor cocktail. The tubules were retained on the mesh Samples were suspended in excess PBS containing protease inhibitor cocktail, and washed and collected by settling 3 times. Washed samples were examined by histology using staining with Sirius red, and by immunohistology to examine the presence of basement membrane components using antibodies to laminin and collagen type IV.

### Example 5 Alternative isolation of basement membrane from kidney

Fresh bovine kidney was obtained from an abattoir. After removal of the external membranes, the tissue comprising the glomerulus was sliced into approximately 5 x 5 x 5mm pieces and treated according to the procedure given in Example 4.

### Example 6 Alternative isolation of basement membrane from lung

Fresh bovine lung was obtained from an abattoir. After removal of extraneous tissues including the main bronchial tubes, the tissue comprising mainly the alveolar tissue was sliced into approximately 5 x 5 x 5mm pieces and treated for isolation of basement membrane particles according to the procedure given in Example 4.

### Example 7 Isolation of basement membrane from tissues

Fresh testis, kidney or lung tissue was obtained from an abattoir and prepared into 5 x 5x 5mm pieces as described in examples 1, 2, 3. Basement membrane tissue was prepared as described in Example 4, except that 0.1% Triton X-100 (or 1% sodium deoxycholoate or 25mM N-octyl glucoside) was added to the PBS and protease cocktail solutions. In the two final washes, the detergent was omitted.

### Example 8 Isolation of basement membrane from muscle

Fresh bovine skeletal muscle was obtained from an abattoir. After removal of any fat and fibrous tissues, the tissue was sliced into approximately 8 x 8 x 8mm pieces and treated by immersion in 2.5% glutaraldehyde in 100 mM neutral phosphate buffer, pH 7.3 for 80 h. The fixed tissue was then soaked without stirring in 10% w/v NaOH. The fixed, alkali treated tissue was then immersed in excess water which was changed every 24 h for 1 week, while avoiding any harsh mechanical treatment. The intact, cross-linked basement membrane tissue, which formed an open sponge-like material was examined by scanning electron microscopy and histology.

### Example 9 Derivation of basement membrane particles

Basement membrane samples, isolated by, for example, one of the methods given in Examples 1-8, were fragmented further using mechanical action by using a Ultra Turrax blender (IKA Werke, Germany). The resulting suspension was examined by microscopy to determine the particle size that was present. The particles were observed to have a maximal dimension of 500 µm. See Figure 2.

Example 10 Alternative derivation of basement membrane particles Basement membrane samples, isolated by, for example, one of the methods given in Examples 1-8, were fragmented further by freezing in liquid nitrogen and powdered by mechanical action, such as, or equivalent to a pestle and mortar. After evaporation of the liquid nitrogen, the particles were brought to room temperature in a dry environment and were examined by microscopy to determine the particle size that was present. The particles were observed to have a maximal dimension of 500 µm.

### Example 11 Further alternative derivation of basement membrane particles

Basement membrane samples, isolated by, for example, one of the methods given in Examples 1-8, were fragmented further using mechanical action by using a gas (liquid nitrogen) cavitation device. The resulting particles were examined by microscopy to determine the size of the particles. The particles were observed to have a maximal dimension of 500 µm.

### Example 12 Fractionation of basement membrane particles

The basement membrane particles, produced by methods such as are described in Examples 9-11, can be fractionated to give particles of the desired size by use of mesh sieves of appropriate size. For example, for the production of particles having an average maximal dimension of about 100 µm; particles may be fractionated by serial passage through a 140 µm sieve and retention of particles by a 70 µm sieve. The consistency of the product can be examined by microscopy.

### Example 13 Alternative fractionation of basement membrane particles

The basement membrane particles, produced by methods such as are described in Examples 9-11, can be fractionated to give particles of the desired size by allowing the particles to settle from excess PBS and selection of aqueous fractions during the settling. Particles can then be collected by brief centrifugation. The consistency of the product can be examined by microscopy.

### Example 14 Culture of spermatogonial stem cells on basement membrane particles

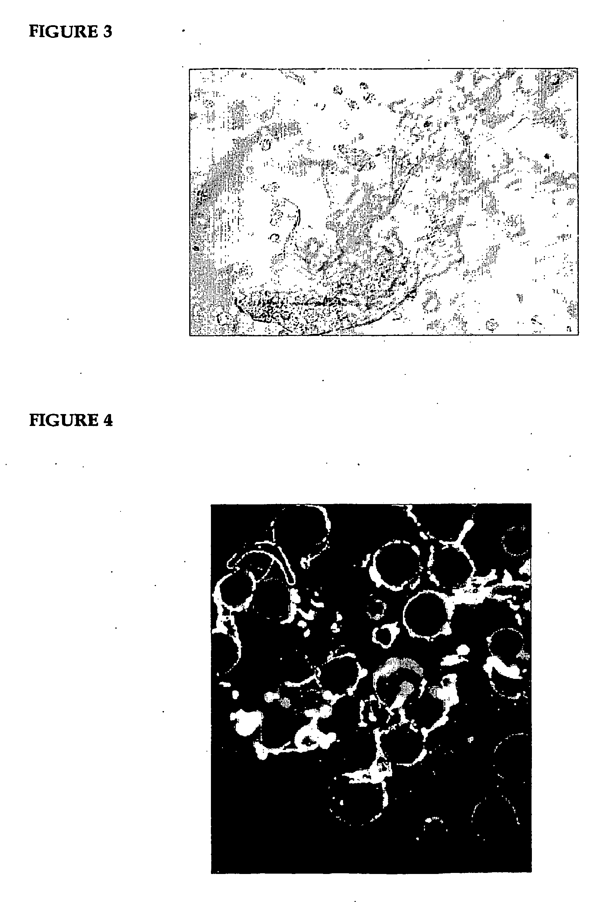

Basement membrane particles, prepared in accordance with methods given in examples 12-13, were used as cell culture substrates. The average maximal dimension of the particles was 100 µm. A preparation, enriched in spermatogonial stem cells from fresh bovine testis in DMEM/F12-10% foetal calf serum (Gibco, USA; JRH Biosciences, USA) containing 1% penicillin (Gibco, USA) and streptomycin (Gibco, USA) was added to the particles and cells were allowed to attach for 4 hours, with intermitted stirring. The seeded particles were then cultured in a continuous spinner culture system (Techne, USA), using an initial density of 5 x 10⁵ cells/150 mg particles in 50ml medium. See Figure 3.

### Example 15 Culture of Sertoli cells on basement membrane particles

Basement membrane particles, prepared according to methods given in examples 12-13, were used as cell culture substrates. The average maximal dimension of the particles was 100 µm. A preparation of Sertoli cells from fresh bovine testis or as cell line TM4 (American Tissue Culture Collection; CRL-1715) in DMEM/F12-10% foetal calf serum containing 1 % penicillin (Gibco, USA) and streptomycin (Gibco, USA) was added to the particles and cells were allowed to attach for 4 hours, with intermitted stirring. The seeded particles were then cultured in a continuous spinner culture system as described in Example 14.

### Example 16 Culture of endothelial cells on basement membrane particles

Basement membrane particles, prepared according to methods given in examples 12-13, were used as cell culture substrates. The average maximal dimension of the particles was 100 µm. A preparation of endothelial cells isolated from human umbilical vein in M199 medium (Gibco, USA) and 10% foetal calf serum JRH Biosciences, USA) containing 1% penicillin and streptomycin and ECGS (endothelial cell growth supplement, 60 µg/ml) (ECGS; Sigma, USA) was added to the particles and cells were allowed to attach for 4 hours, with intermitted stirring. The seeded particles were then cultured in a continuous spinner culture system, as described in Example 14.

### Example 17 Culture of mesenchymal cells on basement membrane particles

Basement membrane particles, prepared according to methods given in examples 12-13, were used as cell culture substrates. The average maximal dimension of the particles was 100 µm. A preparation of mesenchymal stem cells in DMEM/F12-10% foetal calf serum containing 1% penicillin and streptomycin was added to the particles and cells were allowed to attach for 4 hours, with intermitted stirring. The seeded particles were then cultured in a continuous spinner culture system, as described in Example 14.

### Example 18 Production of a basement membrane-like substrate on beads using endothelial cells

Endothelial cells, derived from human umbilical vein were added to 175 µm Cytodex 3 beads (Amersham Pharmacia Biotech, Sweden) and allowed to attach for 4 hours with intermittent stirring. The bead-bound cells were then cultured in a continuous spinner culture system in M199 medium (Gibco, USA) and 10% foetal calf serum containing 1% penicillin and streptomycin and ECGS (endothelial cell growth supplement, 60 µg/ml) (Sigma) with daily addition of sodium ascorbate to a final concentration of 0.05 mg/ml. After 5 days the beads, which were now coated in a basement membrane-like substrate were isolated, and the cells detached by treatment with dilute ammonia solution. Optionally, the basement membrane-like substrate was stabilised by treatment with 0.1% glutaraldehyde or other fixation system. See Figure 4.

### Example 19 Production of a basement membrane-like substrate on beads using Sertoli cells

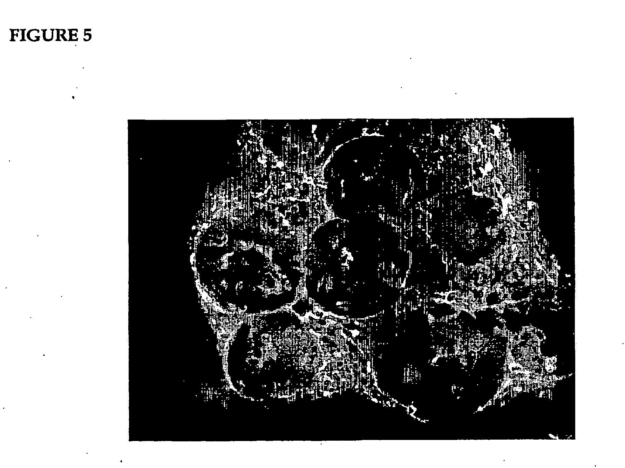

Sertoli cells, as cell line TM4 (American Tissue Culture Collection; CRL-1715) were added to 260 µm Cultispher-S beads (range 130 to 380 µm) (Sigma, USA) and allowed to attach for 4 hours with intermittent stirring. The bead-bound cells were then cultured in a continuous spinner culture system in DMEM-F12 containing 10% foetal calf serum containing 1% penicillin and streptomycin with daily addition of sodium ascorbate. After 5 days, the beads, which were now coated in a basement membrane-like substrate, were isolated and the cells detached by treatment with dilute ammonia solution. Optionally, the basement membrane-like substrate was stabilised by treatment with 0.1% glutaraldehyde or other fixation system. See Figure 5.

### Example 20 Production of a basement membrane-like substrate on resorbable beads using Sertoli cells

Sertoli cells, as cell line TM4 (American Tissue Culture Collections; CRL-1715) were added to PLGA beads. PLGA beads were made by an emulsion method. That is, a 10% (w/v) solution of PLGA in dichloromethane was emulsified into an aqueous solution containing 1% (w/v) poly(vinyl alcohol) by stirring. PLGA beads where allowed to settle, and were washed 5 times with water by decanting. The round-shaped, transparent beads were finally fractionated by using sieves with a size of 47 µm and 210 µm, respectively. The average maximal dimension of the sieved PLGA beads was about 125 µm (range 47 to 210 µm). Cells were allowed to attach for 4 hours with intermitted stirring. The bead-bound cells were then cultured in a continuous spinner culture system in DMEM-F12 containing 10% foetal calf serum containing 1% penicillin and streptomycin with daily addition of sodium ascorbate. After 5 days, the beads, which were now coated in basement membrane-like substrate, were isolated and the cells detached by treatment with dilute ammonia solution. Optionally, the basement membrane-like substrate was stabilised by treatment with 0.1 % glutaraldehyde or other fixation system.

### Example 21 Production of a basement membrane-like substrate on beads using osteoblasts

Osteoblast cells, either as primary isolates from bone marrow or as an established cell line, were added to 260 µm Cultispher-S beads (range 130 to 380 µm) (Sigma, USA) and allowed to attach for 4 hours with intermittent stirring. The bead-bound cells were then cultured in a continuous spinner culture system in DMEM-F12 containing 10 % foetal calf serum containing 1% penicillin and streptomycin with daily addition of sodium ascorbate. After 5 days, the beads, which were now coated in basement membrane-like substrate, were isolated and the cells detached by treatment with dilute ammonia solution. Optionally, the basement membrane-like substrate was stabilised by treatment with 0.1% glutaraldehyde or other fixation system.

### Example 22 Growth of spermatogonial stem cells on beads coated with a basement membrane-like substrate

Spermatogonial stem cells, isolated from fresh bovine testis, were seeded onto Cultispher beads coated with a basement membrane-like substrate, prepared according to Example 19, and cultured in a continuous spinner culture system in DMEM-F12 containing 10% foetal calf serum containing 1% penicillin and streptomycin. Cell growth was monitored by microscopy.

### Example 23 Growth of haematopoietic stem cells on beads coated with a basement membrane-like substrate

Haematopoietic stem cells (HSC), isolated from fresh human bone marrow, were seeded onto Cultispher beads coated with a basement membrane-like substrate, prepared according to Example 21, and cultured in a continuous spinner culture system in DMEM-F12 containing 10% foetal calf serum containing 1% penicillin and streptomycin. Cell growth was monitored by microscopy.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

All publications mentioned in this specification are herein incorporated by reference. Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed in Australia or elsewhere before the priority date of each claim of this application.

It will be appreciated by those skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

### REFERENCES

1. Brinster, RL et al., Spermatogenesis following male germ-cell transplantation, The Proceedings of the National Academy of Science USA, 91:11298-11302 (1994)
2. Enders, GC et al., Sertoli Cell Binding to Isolated Testicular Basement Membrane, The Journal of Cell Biology, 103:1109-1119 (1986)
3. Ogawa, T et al., Transplantation of male germ cell line stem cells restores fertility in infertile mice, Nature Medicine, 5:29-34 (1999)
4. van der Wee, K and Hofmann, MC, An in vitro Tubule Assay Identifies HGF as a Morphogen for the Formation of Seminiferous. Tubules in the Postnatal Mouse Testis, Experimental Cell Research, 252:175-185 (1999)

## Claims

1. A method for supporting the *in vitro* growth of one or more eukaryotic cell type(s), said method comprising;
seeding cells of said cell type(s) onto particles, and
culturing said seeded cells *in vitro* under conditions suitable for expansion of said seeded cells,
**characterised in that** said particles are less than about 500 µm in size and comprise fragments of intact natural basement membrane maintaining the structure of said basement membrane, or fragments of a basement membrane-like substrate produced In culture by basement membrane-synthesising cells and consisting of a network of ordered interacting molecules consisting of at least collagen IV and laminin and having a structure analogous to intact natural basement membrane.

2. The method of claim 1, wherein the particles have an average size in the range of from about 50 µm to 400 µm.

3. The method of claim 1, wherein the particles have an average size of about 100 µm.

4. The method of any one of claims 1 to 3, wherein the particles comprise fragments of intact natural basement membrane maintaining the structure of said basement membrane.

5. The method of claim 4, wherein the natural basement membrane is derived from seminiferous tubules.

6. The method of claim 4, wherein the natural basement membrane is derived from tissue selected from the group consisting of blood vessels, perivascular tissue, placenta, lung, skin and tissue of bone marrow or the bone/bone marrow interface.

7. The method of any one of claims 1 to 3, wherein the particles comprise fragments of the basement membrane-like substrate produced in culture by basement membrane-synthesizing cells and consisting of a network of ordered interacting molecules consisting of at least collagen type IV and laminin and having a structure analogous to intact natural basement membrane.

8. The method of any one of claims 1 to 3 or 7, wherein the method further comprises preparing the particles onto which the said cells are seeded, the preparation comprising attaching onto suitable beads, basement membrane-synthesising cells and culturing the bead-bound cells under conditions suitable for the expression and secretion of basement membrane constituents to thereby produce particles comprising beads coated, at least partially, in the basement membrane-like substrate.

9. The method of claim 8, wherein the basement membrane-synthesising cells are destroyed or removed from the particles before the said cells are seeded.

10. The method of any one of claims 7 to 9, wherein the basement membrane-like substrate is stabilised.

11. The method of claim 7 or 8, wherein the basement membrane-synthesising cells are co-cultured on beads with the cells to be grown.

12. The method of any one of claims 7 to 11, wherein the basement membrane-synthesising cells are selected from the group consisting of endothelial cells, keratinocytes, Schwann cells, Sertoli cells, osteoblasts and combinations thereof.

13. The method of any one of claims 1 to 12, wherein the seeded cells are selected from the group consisting of endothelial cells, skin fibroblasts, myoblasts and cardiomyoblasts.

14. The method of any one of claims 1 to 12, wherein the seeded cells are selected from the group consisting of embryonic stem cells, adult mesenchymal stem cells, and cord blood cells.

15. The method of any one of claims 8 to 11, wherein the basement membrane-synthesising cells are Sertoli cells and the seeded cells are spermatogonial stem cells.

16. The method of any one of claims 8 to 11, wherein the basement membrane-synthesising cells are osteoblasts and the seeded cells are haematopoietic stem cells.

17. A preparation of particles comprising coated beads **characterised in that** the beads are coated, at least partially, in fragments of a basement membrane-like substrate consisting of a network of ordered interacting molecules consisting of at least collagen IV and laminin and having a structure analogous to intact natural basement membrane, wherein said particles are less than about 500 µm In size.

18. The preparation of claim 17, wherein the particles have an average size in the range of from about 50 µm to 400 µm.

19. The preparation of claim 17 or 18, wherein the particles have an average size of about 100 µm.

20. The preparation of any one of claims 17 to 19, wherein said particles are prepared by a process comprising attaching onto suitable beads, basement membrane-synthesising cells and culturing the bead-bound cells under conditions suitable for the expression and secretion of basement membrane proteins.

21. The preparation of claim 20, wherein the basement membrane-synthesising cells are selected from the group consisting of endothelial cells, keratinocytes, Schwann cells, Sertoli cells, osteoblasts and combinations thereof.

22. A preparation of particles **characterised in that** the particles comprise fragments of intact natural basement membrane maintaining the structure of said basement membrane, wherein said particles are less than about 500 µm in size.

23. The preparation of claim 22, wherein the particles have an average size in the range of from about 50 µm to 400 µm.

24. The preparation of claim 22 or 23, wherein the particles have an average size of about 100 µm.

25. The preparation of any one of claims 22 to 24, wherein the fragments of intact natural basement membrane are derived from seminiferous tubules.

26. The preparation of any one of claims 22 to 24, wherein the fragments of intact natural basement membrane are derived from tissue selected from the group consisting of blood vessels, perivascular tissue, placenta, lung, skin and tissue of bone marrow or the bone/bone marrow interface.

## Patentansprüche

1. Verfahren zur Unterstützung des In-vitro-Wachstums eines oder mehrerer eukaryotischer Zelltypen, wobei das Verfahren Folgendes umfasst:
das Beimpfen von Zellen des Zelltyps/der Zelltypen auf Partikel sowie
das Züchten der beimpften Zellen in vitro unter Bedingungen, die für die Vermehrung der beimpften Zellen geeignet sind,
**dadurch gekennzeichnet, dass** die Partikel weniger als etwa 500 µm groß sind und Fragmente intakter natürlicher Basalmembranen, die die Struktur der Basalmembran beibehalten, oder Fragmente eines Basalmembran-artigen Substrats umfassen, die in Kultur durch Basalmembran-synthetisierende Zellen produziert werden und aus einem Netzwerk geordneter, in Wechselwirkung stehender Moleküle bestehen, die zumindest aus Kollagen-IV und Laminin bestehen und eine Struktur aufweisen, die analog zu intakter natürlicher Basalmembran ist.

2. Verfahren nach Anspruch 1, worin die Partikel eine durchschnittliche Größe im Bereich von etwa 50 µm bis 400 µm aufweisen.

3. Verfahren nach Anspruch 1, worin die Partikel eine durchschnittliche Größe von etwa 100 µm aufweisen.

4. Verfahren nach einem der Ansprüche 1 bis 3, worin die Partikel Fragmente intakter natürlicher Basalmembran umfassen, die die Struktur der Basalmembran beibehalten.

5. Verfahren nach Anspruch 4, worin die natürliche Basalmembran von Hodenkanälchen abstammt.

6. Verfahren nach Anspruch 4, worin die natürliche Basalmembran von Gewebe abstammt, das aus der aus Blutgefäßen, perivaskulären Gefäßen, der Plazenta, der Lunge, der Haut und Gewebe des Knochenmarks oder der Knochen/Knochmark-Grenzfläche bestehenden Gruppe ausgewählt ist.

7. Verfahren nach einem der Ansprüche 1 bis 3, worin die Partikel Fragmente des Basalmembran-artigen Substrats umfassen, die in Kultur durch Basalmembran-synthetisierende Zellen produziert werden und aus einem Netzwerk geordneter, in Wechselwirkung stehender Moleküle bestehen, die zumindest aus Kollagen-Typ-IV und Laminin bestehen und eine Struktur aufweisen, die analog zu intakter natürlicher Basalmembran ist.

8. Verfahren nach einem der Ansprüche 1 bis 3 oder 7, worin das Verfahren weiters das Herstellen der Partikel umfasst, auf die die Zellen beimpft werden, wobei die Herstellung das Binden Basalmembran-synthetisierender Zellen an geeignete Perlen sowie das Züchten der perlengebundenen Zellen unter Bedingungen umfasst, die für die Expression und die Sekretion von Basalmembran-Konstituenten geeignet sind, um **dadurch** Partikel zu produzieren, die Perlen umfassen, die, zumindest teilweise, mit dem Basalmembran-artigen Substrat beschichtet sind.

9. Verfahren nach Anspruch 8, worin die Basalmembran-synthetisierenden Zellen zerstört oder aus den Partikeln entfernt werden, bevor die Zellen beimpft werden.

10. Verfahren nach einem der Ansprüche 7 bis 9, worin das Basalmembran-artige Substrat stabilisiert ist.

11. Verfahren nach Anspruch 7 oder 8, worin die Basalmembran-synthetisierenden Zellen auf Perlen mit den zu züchtenden Zellen cokultiviert werden.

12. Verfahren nach einem der Ansprüche 7 bis 11, worin die Basalmembran-synthetisierenden Zellen aus der aus Endothel-Zellen, Keratinozyten, Schwann-Zellen, Sertoli-Zellen, Osteoblasten und Kombinationen dieser bestehenden Gruppe ausgewählt sind.

13. Verfahren nach einem der Ansprüche 1 bis 12, worin die beimpften Zellen aus der aus Endothel-Zellen, Haut-Fibroblasten, Myoblasten und Cardiomyoblasten bestehenden Gruppe ausgewählt sind.

14. Verfahren nach einem der Ansprüche 1 bis 12, worin die beimpften Zellen aus der aus embryonalen Stammzellen, erwachsenen mesenchymalen Stammzellen und Nabelschnurblutzellen bestehenden Gruppe ausgewählt sind.

15. Verfahren nach einem der Ansprüche 8 bis 11, worin die Basalmembran-synthetisierenden Zellen Sertoli-Zellen sind und die beimpften Zellen spermatogoniale Stammzellen sind.

16. Verfahren nach einem der Ansprüche 8 bis 11, worin die Basalmembran-synthetisierenden Zellen Osteoblasten sind und die beimpften Zellen hämatopoetische Stammzellen sind.

17. Präparat aus Partikeln, umfassend beschichtete Perlen, **dadurch gekennzeichnet, dass** die Perlen, zumindest teilweise, mit Fragmenten eines Basalmembran-artigen Substrats beschichtet sind, die aus einem Netzwerk an geordneten, in Wechselwirkung stehenden Molekülen bestehen, die zumindest aus Kollagen-IV und Laminin bestehen und eine Struktur aufweisen, die analog zu intakter natürlicher Basalmembran ist, worin die Partikel weniger als etwa 500 µm groß sind.

18. Präparat nach Anspruch 17, worin die Partikel eine durchschnittliche Größe im Bereich von etwa 50 µm bis 400 µm aufweisen.

19. Präparat nach Anspruch 17 oder 18, worin die Partikel eine durchschnittliche Größe von etwa 100 µm aufweisen.

20. Präparat nach einem der Ansprüche 17 bis 19, worin die Partikel durch ein Verfahren hergestellt werden, das die Bindung Basalmembran-synthetisierender Zellen an geeignete Perlen sowie das Züchten der perlengebundenen Zellen unter Bedingungen umfasst, die für die Expression und die Sekretion von Basalmembran-Proteinen geeignet sind.

21. Präparat nach Anspruch 20, worin die Basalmembran-synthetisierenden Zellen aus der aus Endothel-Zellen, Keratinozyten, Schwann-Zellen, Sertoli-Zellen, Osteoblasten und Kombinationen dieser bestehenden Gruppe ausgewählt sind.

22. Präparat aus Partikeln, **dadurch gekennzeichnet, dass** die Partikel Fragmente intakter natürlicher Basalmembran umfassen, die die Struktur der Basalmembran beibehalten, worin die Partikel weniger als etwa 500 µm groß sind.

23. Präparat nach Anspruch 22, worin die Partikel eine durchschnittliche Größe im Bereich von etwa 50 µm bis 400 µm aufweisen.

24. Präparat nach Anspruch 22 oder 23, worin die Partikel eine durchschnittliche Größe von etwa 100 µm aufweisen.

25. Präparat nach einem der Ansprüche 22 bis 24, worin die Fragmente intakter natürlicher Basalmembran von Hodenkanälchen abstammen.

26. Präparat nach einem der Ansprüche 22 bis 24, worin die Fragmente intakter, natürlicher Basalmembran von Gewebe abstammen, das aus der aus Blutgefäßen, perivaskulären Gefäßen, der Plazenta, der Lunge, der Haut und Gewebe des Knochenmarks oder der Knochen/Knochmark-Grenzfläche bestehenden Gruppe ausgewählt ist.

## Revendications

1. Procédé pour supporter la croissance *in vitro* d'un ou plusieurs types de cellule eucaryote, ledit procédé comprenant les étapes consistant à:
ensemencer des cellules dudit ou desdits types de cellule sur des particules, et
mettre en culture lesdites cellules ensemencées *in vitro* dans des conditions appropriées pour un développement desdites cellules ensemencées,
**caractérisé en ce que** lesdites particules ont une taille inférieure à environ 500 µm et comprennent des fragments de membrane basale naturelle maintenant la structure de ladite membrane basale, ou des fragments d'un substrat de type membrane basale produit en culture par des cellules de synthèse de membrane basale et constitué d'un réseau de molécules en interaction ordonnée constituées d'au moins le collagène IV et la laminine et ayant une structure analogue à une membrane basale naturelle intacte.

2. Procédé selon la revendication 1, dans lequel les particules ont une taille moyenne dans la plage de 50 µm à 400 µm.

3. Procédé selon la revendication 1, dans lequel les particules ont une taille moyenne d'environ 100 µm.

4. Procédé selon l'une quelconque des revendications 1 à 3, dans lequel les particules comprennent des fragments de membrane basale naturelle intacte maintenant la structure de ladite membrane basale.

5. Procédé selon la revendication 4, dans lequel la membrane basale naturelle est dérivée de tubes séminifères.

6. Procédé selon la revendication 4, dans lequel la membrane basale naturelle est dérivée d'un tissu choisi dans le groupe constitué par les vaisseaux sanguins, le tissu périvasculaire, le placenta, le poumon, la peau et le tissu de moelle osseuse ou l'interface os/moelle osseuse.

7. Procédé selon l'une quelconque des revendications 1 à 3, dans lequel les particules comprennent des fragments du substrat de type membrane basale produit en culture par des cellules de synthèse de membrane basale et constitué d'un réseau de molécules en interaction ordonnée constituées d'au moins le collagène de type IV et la laminine et ayant une structure analogue à une membrane basale naturelle intacte.

8. Procédé selon l'une quelconque des revendications 1 à 3 ou 7, dans lequel le procédé comprend en outre la préparation de particules sur lesquelles lesdites cellules sont ensemencées, la préparation comprenant la fixation sur des billes appropriées, de cellules de synthèse de membrane basale et la mise en culture de cellules liées aux billes dans des conditions appropriées pour l'expression et la sécrétion de constituants de membrane basale pour ainsi produire des particules comprenant des billes revêtues, au moins partiellement, dans le substrat de type membrane basale.

9. Procédé selon la revendication 8, dans lequel les cellules de synthèse de membrane basale sont détruites ou éliminées des particules avant l'ensemencement desdites cellules.

10. Procédé selon l'une quelconque des revendications 7 à 9, dans lequel le substrat de type membrane basale est stabilisé.

11. Procédé selon la revendication 7 ou 8, dans lequel les cellules de synthèse de membrane basale sont mises en culture conjointe sur des billes avec les cellules à faire croître.

12. Procédé selon l'une quelconque des revendications 7 à 11, dans lequel les cellules de synthèse de membrane basale sont choisies dans le groupe constitué par les cellules endothéliales, les kératinocytes, les cellules de Schwann, les cellules de Sertoli, les ostéoblastes, et leurs combinaisons.

13. Procédé selon l'une quelconque des revendications 1 à 12, dans lequel les cellules ensemencées sont choisies dans le groupe constitué par les cellules endothéliales, les fibroblastes cutanés, les myoblastes et les cardiomyoblastes.

14. Procédé selon l'une quelconque des revendications 1 à 12, dans lequel les cellules ensemencées sont choisies dans le groupe constitué par les cellules souches embryonnaires, les cellules souches mésenchymateuses adultes, et les cellules sanguines du cordon ombilical.

15. Procédé selon l'une quelconque des revendications 8 à 11, dans lequel les cellules de synthèse de membrane basale sont des cellules de Sertoli et les cellules ensemencées sont des cellules souches spermatogoniales.

16. Procédé selon l'une quelconque des revendications 8 à 11, dans lequel les cellules de synthèse de membrane basale sont des ostéoblastes et les cellules ensemencées sont des cellules souches hématopoïétiques.

17. Préparation de particules comprenant des billes revêtues **caractérisée en ce que** les billes sont revêtues, au moins partiellement, dans des fragments d'un substrat de type membrane basale constitué d'un réseau de molécules en interaction ordonnée constituées d'au moins le collagène IV et la laminine et ayant une structure analogue à une membrane basale naturelle intacte, dans laquelle lesdites particules ont une taille inférieure à 500 µm.

18. Préparation selon la revendication 17, dans laquelle les particules ont une taille moyenne dans la plage d'environ 50 µm à 400 µm.

19. Préparation selon la revendication 17 ou 18, dans laquelle les particules ont une taille moyenne d'environ 100 µm.

20. Préparation selon l'une quelconque des revendications 17 à 19, dans laquelle lesdites particules sont préparées par un procédé comprenant la fixation sur des billes appropriées de cellules de synthèse de membrane basale et la mise en culture de cellules liées aux billes dans des conditions appropriées pour l'expression et la sécrétion de protéines de membrane basale.

21. Préparation selon la revendication 20, dans laquelle les cellules de synthèse de membrane basale sont choisies dans le groupe constitué par les cellules endothéliales, les kératinocytes, les cellules de Schwann, les cellules de Sertoli, les ostéoblastes, et leurs combinaisons.

22. Préparation de particules **caractérisée en ce que** les particules comprennent des fragments de membrane basale naturelle intacte maintenant la structure de ladite membrane basale, dans laquelle lesdites particules ont une taille inférieure à environ 500 µm.

23. Préparation selon la revendication 22, dans laquelle les particules ont une taille moyenne dans la plage d'environ 50 µm à 400 µm.

24. Préparation selon la revendication 22 ou 23, dans laquelle les particules ont une taille moyenne d'environ 100 µm.

25. Préparation selon l'une quelconque des revendications 22 à 24, dans laquelle les fragments de la membrane basale naturelle intacte sont dérivés de tubes séminifères.

26. Préparation selon l'une quelconque des revendications 22 à 24, dans laquelle les fragments de membrane basale naturelle intacte sont dérivés d'un tissu choisi dans le groupe constitué par les vaisseaux sanguins, le tissu périvasculaire, le placenta, le poumon, la peau et le tissu de moelle osseuse ou l'interface os/moelle osseuse.
